# EUROPEAN PATENT APPLICATION

(11) **EP 1 495 923 A1**
(43) Date of publication of application: **12.01.2005**
(21) Application number: 03745919.5
(22) Date of filing: 04.04.2003
(51) Int. Cl.: B60R 21/32, B60N 2/44

(54) **AIR BAG START CONTROL DEVICE OF VEHICLE**

(30) Priority: 09.04.2002 JP 2002106154
(71) Applicant: M.I.Laboratories Corporation, Tokyo 141-0001 (JP)
(72) Inventor: TAKASHIMA, Mitsuru, M.I.Laboratories Corporation, Tokyo 141-0001 (JP)
(74) Representative: Turner, James Arthur
(86) International application number: PCT/JP2003/004344
(87) International publication number: WO 2003/084786

(57) **Abstract**

An air bag start control device of vehicle capable of optimally controlling the operation of an air bag by determining whether a person is seated on the vehicle seat or not and, when seated, by determining the body form thereof, wherein an air bladder type pressure sensor is fitted to either or both of the back rest and seat part of the vehicle seat to control the amount and the direction of inflation of the air bag according to whether or not living body signals such as respiration and pulsation are included in electric outputs from the pressure sensors, when included, according to whether the outputs exceed specified limits or not.

## Description

### Technical Field

This invention relates to a vehicular airbag control system for controlling such performance parameters of an airbag as triggering or non-triggering thereof, and the force and direction of airbag inflation, using a pneumatic pressure sensor or sensors installed in a vehicle seat for determination of the specifics of the particular occupant of the seat.

### Background Art

Airbags have found widespread use on motor vehicles for safeguarding the drivers and passengers against traffic accidents by mitigating the impacts of collision. Mounted in front of or on the sides of the vehicle seats, airbags are sealed hermetically and each furnished with an inflation device including a solid propellant. The propellant is detonated in the event of an abrupt change in vehicle acceleration due to a collision. The gas created by the detonated propellant instantly inflates the bag thereby causing the same to keep the occupant of the seat confmed thereto against the momentum of the collision.

As heretofore constructed, however, a great majority of airbags have had their performance predetermined in terms of bag volume upon inflation, the angle of deployment and so forth in consideration of average size adults. Little or no attention has been paid in most cases to the specific figures and weights of the individual seat occupants. The airbags of such average design could inflict excessive impact upon infants and small children when deployed, sometimes hurting them nearly as seriously as the collision itself.

An additional inconvenience heretofore encountered with the airbags is that they could be triggered off even when the seats were unoccupied or when luggage was placed thereon. Inflated unnecessarily, the airbags could send such luggage hurtling off the seats. Both driver and passengers might also suffer from a sudden pressure change caused inside the vehicle by the unnecessarily inflated airbags. The airbags should therefore be prevented from deployment when the associated seats are unoccupied or merely occupied by luggage.

### Disclosure of the Invention

The present invention provides an airbag control system having a pneumatic pressure sensor or sensors mounted to each vehicle seat in order to ascertain whether the seat is occupied or not. Luggage is discriminated from humans if the sensor output signal or signals have no sustained periodicity indicative of such rhythmic bodily processes as pulsation and respiration, which are distinguishable from the bumps and jolts of the vehicle. If the sensor output signal or signals represent the rhythmic bodily processes, on the other hand, then the body size of the seat occupant is determined on the basis of the signal magnitude.

All the foregoing considerations lead to optimal control of the airbags depending upon the body size of the seat occupants and other factors. Optionally, the airbag control system according to the invention may be made active only during vehicle travel as ascertained from information contained in the sensor output signals above or from some other source on the vehicle.

### Brief Description of the Drawings

Figure 1 is a perspective view of a vehicle seat fitted with pneumatic pressure sensors used in the airbag control system according to the invention.

Figure 2 is an enlarged perspective view of each pressure sensor of Figure 1.

Figure 3 is a graph plotting the curve of the pressure sensor output when a human sits on the vehicle seat, the sensor output here being indicative of human respiration.

Figure 4 is a graph of the output from the pressure sensor on the seat back when an adult of average body size is seated motionlessly while the vehicle is at a standstill, the seat-back pressure sensor output being shown after being processed by a waveform analyzer.

Figure 5 is a graph of the output from the pressure sensor on the seat bottom when the vehicle is traveling with the seat unoccupied, the seat-bottom pressure sensor output being shown after being processed by a waveform analyzer.

Figure 6 is a graph of the output from the seat-bottom pressure sensor when the vehicle is traveling with the seat loaded with luggage, the seat-bottom pressure sensor output being shown after being processed by a waveform analyzer.

Figure 7 is a graph of the output from the seat-bottom pressure sensor when the vehicle is traveling with the seat occupied by a human, the seat-bottom pressure sensor output being shown after being processed by a waveform analyzer.

Figure 8 is a block diagram of the airbag control system according to the invention for optimizing the performance of the airbag by detecting the human or luggage on the seat from the seat-back and seat-bottom pressure sensor outputs.

Figure 9 is an illustration of how the airbag is inflated under the direction of the airbag control system.

### Best Mode of Carrying Out the Invention

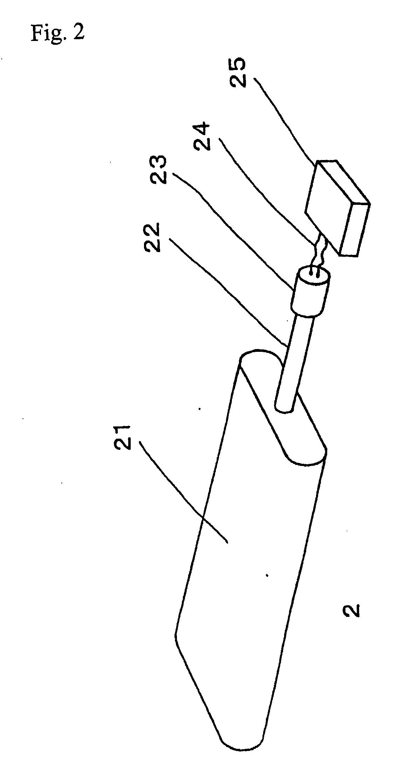

The present invention will now be described in terms of the illustrated embodiment thereof. The pneumatic pressure sensors for use in the practice of the invention may each be constructed as depicted in Figure 2 and therein generally designated 2. The pressure sensor 2 includes a pneumatic sensing bag 21 of relatively thin, elongate boxlike shape fabricated from rubber or like pliant plastics. The sensing bag 21 need not be perfectly airtight; rather, for ease of mass production and immunity from ambient temperature changes, it may have pinholes, permitting some slight inflow and outflow of air. The sensing bag 21 should not, however, be allowed to get flattened out but should retain its original shape as long as possible, as by being stuffed with sponge or the like.

Communicatively joined to one end of the sensing bag 21 is a conduit 22 leading to a microphone 23. Pressure variations created inside the sensing bag 21 are therefore conveyed via the conduit 22 to the diaphragm of the microphone 23. The electric output from the microphone 23 is sent over conductors 24 to an electric circuit 25 thereby to be processed as required preparatory to transmission, either by radio or over wires.

The microphone 23, conductors 24 and electric circuit 25 may all be combined into one-piece construction by being accommodated in a single housing. It is also possible to install the microphone 23 inside the sensing bag 21, thereby forgoing the conduit 22.

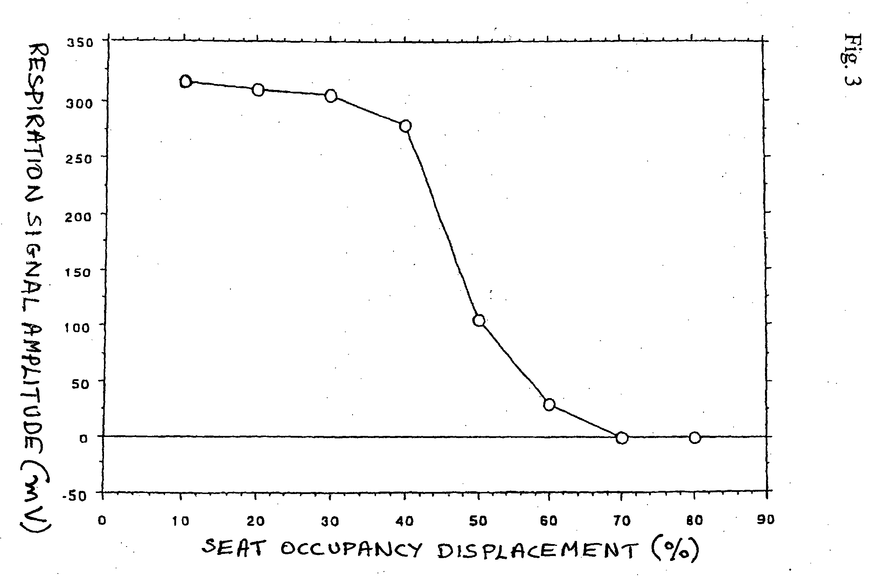

Figure 3 graphically indicates the amplitude of the respiration signal produced by the electric circuit 25 when a human sits on the vehicle seat in various positions with respect to the pressure sensor 2. The vertical axis of the graph represents the amplitude in millivolts (mV) of the respiration signal generated by the pressure sensor 2, whereas the horizontal axis represents the percentage of seat occupant displacement with respect to the sensing bag of the pressure sensor. The occupant displacement percentage is calculated by first subtracting the area of that part of the sensing bag surface which is actually occupied by the driver or passenger, from the total area of the sensing bag surface, then by dividing the difference by the total area of the sensing bag surface, and then by multiplying the result of the division by 100.

A closer study of the graph will now prove that the pressure sensor output magnitude is substantially constant when the occupant displacement percentage is up to 30, abruptly drops above that value, and becomes zero when the percentage rises to 70. This sudden drop in signal strength may be explained as follows:

When the occupant displacement percentage becomes so high that only one half or so of the sensing bag is weighed down by the seat occupant, the other, unloaded part of the sensing bag starts absorbing very substantive part of the internal pressure variations caused by the respiration or other bodily motion of the occupant. Thus, it is reasoned, the sensing bag fails to convey a corresponding proportion of its internal pressure changes to the microphone 23. The present invention takes positive advantage of this sensing loss of the sensing bag on the vehicle seat for controlling the performance of the associated airbag or airbags.

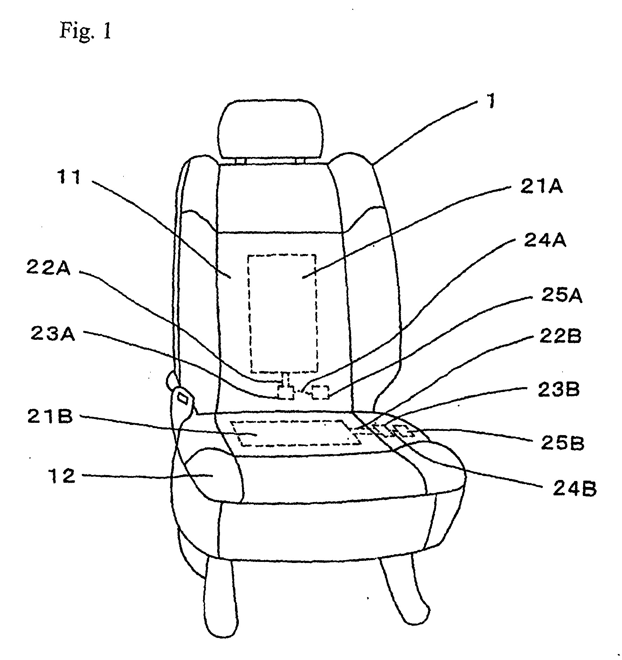

As illustrated in Figure 1, each vehicle seat may be furnished with two pneumatic pressure sensors each constructed as set forth above with reference to Figure 2. The vehicle seat 1 has the sensing bag 21*A* of one pressure sensor mounted centrally and internally of its back 11. This sensing bag 21*A* should be sufficiently large to cover most of the back of the seated adult, typically about 50 centimeters in vertical dimension and about 30 centimeters in horizontal dimension. The seat-back pressure sensor additionally comprises the conduit 22*A*, microphone 23*A*, conductors 24*A* and electric circuit 25*A*. The electric circuit 25*A* sends the seat-back sensor signal *A* by radio or over wires.

Another pressure sensor has its sensing bag 21*B* mounted centrally and internally of the bottom 12 of the vehicle seat 1. The sensing bag 21*B* of this seat-bottom pressure sensor should be large enough to cover the thighs of an adult, typically about 40 centimeters in vehicle side-to-side transverse dimension and about 25 centimeters in vehicle front-to-rear depth dimension. The seat-bottom pressure sensor additionally comprises the conduit 22*B*, microphone 23*B*, conductors 24*B* and electric circuit 25*B*. The electric circuit 25*B* transmits the seat-bottom sensor signal *B* by radio or over wires.

The outputs *A* and *B* from both seat-back pressure sensor and seat-bottom pressure sensor are at zero level when the vehicle is standing still and, at the same time, when the seat is empty or has luggage placed thereon.

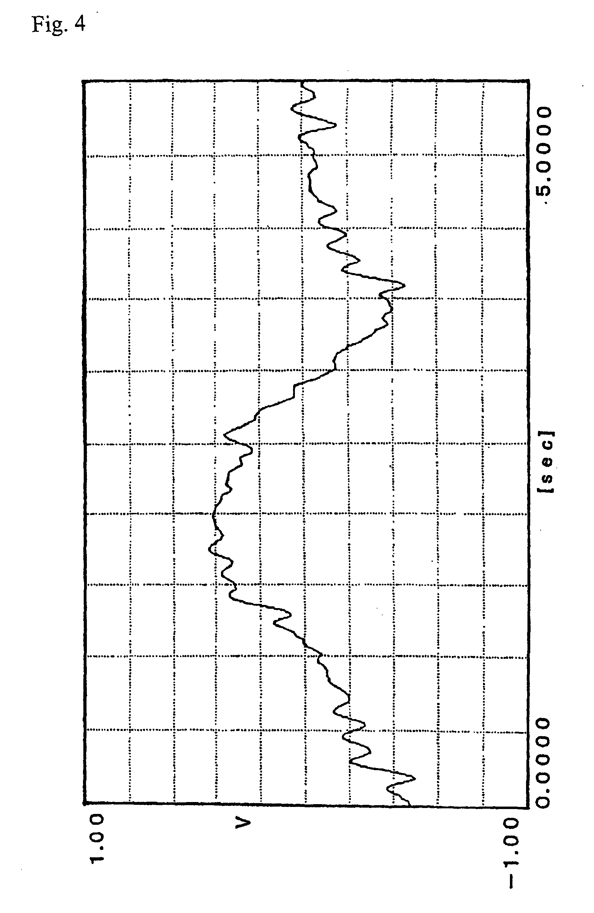

Figure 4 graphically represents the result of waveform analysis of the seat-back pressure sensor output *A* when the vehicle is standing still and, at the same time, when an average-size adult sits motionlessly on the seat. The vertical axis of the graph represents the sensor output magnitude in volts, and the horizontal axis represents time in seconds. It will be noted from this graph that the seat-back pressure sensor output contains a nearly sinusoidal waveform, with a cycle of approximately four seconds, indicative of the bodily motion of the seat occupant due to respiration. Superposed on this sinusoidal waveform are smaller undulations due to less pronounced physical movements such as pulsation, which manifest themselves even when the human is seated still.

The output *B* from the seat-bottom pressure sensor under like conditions is analogous in waveform with the seat-back pressure sensor output *A.* There does, however, exist a difference in signal magnitude by reason of the difference in cushioning between seat back 11 and seat bottom 12.

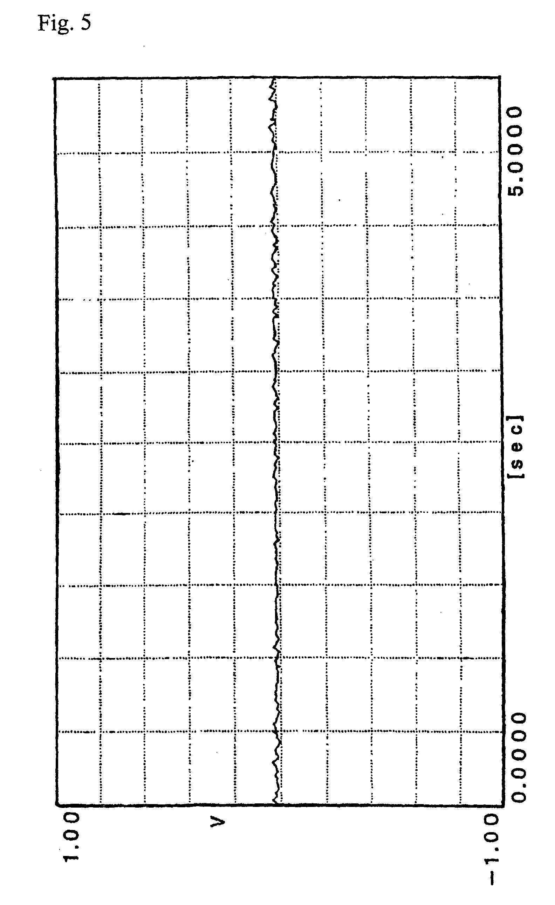

Figure 5 is a graphic representation of the waveform-analyzed seat-bottom pressure sensor output *B* when the vehicle is running with the seat unoccupied. Theoretically, the seat-bottom pressure sensor output level should be zero under the noted conditions, but, actually, minute fluctuations are observable in signal level. Such fluctuations are ascribable to the fluttering of the sensing bag 21*B* due to vehicle vibration, and to the consequent pressure variations inside the bag which are sensible by the microphone. Whatever the reason may be, these minute fluctuations in sensor output level bear no significance and are negligible.

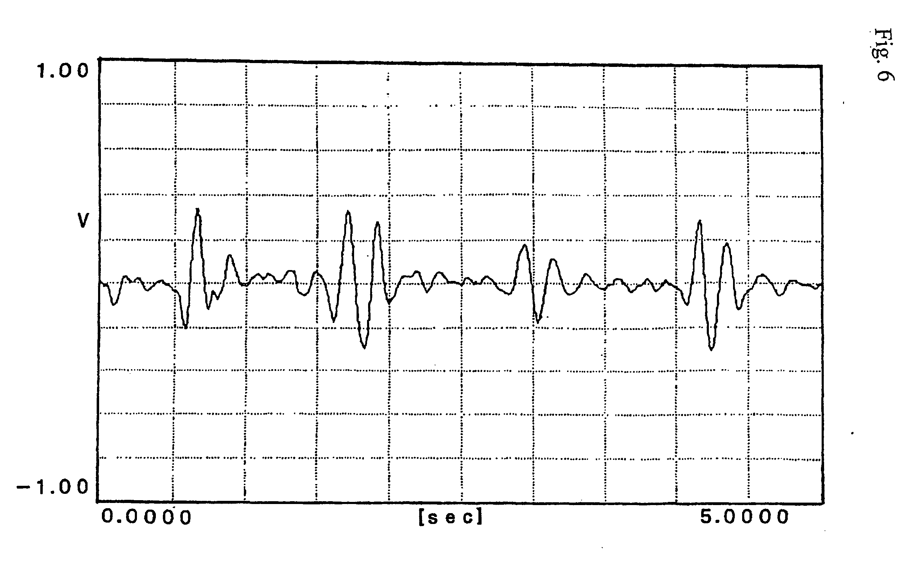

Graphically represented in Figure 6 is the waveform-analyzed seat-bottom pressure sensor output *B* when the vehicle is running with luggage placed on the seat, or more exactly, on the sensing bag 21*B* of the seat-bottom pressure sensor. The luggage used in this experiment was boxlike in shape, sized 30 by 30 by 30 centimeters and weighing approximately five kilograms.

The seat-bottom pressure sensor output *B* contains pulses because of changes in the force of gravity due to vehicle vibration, particularly in the vertical direction. All such pulses are of brief durations. The mean level of the seat-bottom pressure sensor output *B* is zero.

The seat-back pressure sensor output *A* will be approximately zero in level, as is the seat-back pressure sensor output *B* of Figure 6, if the luggage on the seat is out of contact with the sensing bag 21*A* of the seat back pressure sensor or in contact with less than 30 percent of the surface area of the sensing bag 21*A*. On the other hand, if the luggage is in contact with more than 30 percent, especially more than 50 percent, of the surface area of the seat-back sensing bag 21*A*, the seat-back pressure sensor output *A* will be more or less similar in waveform to the seat-back pressure sensor output *B* of Figure 6.

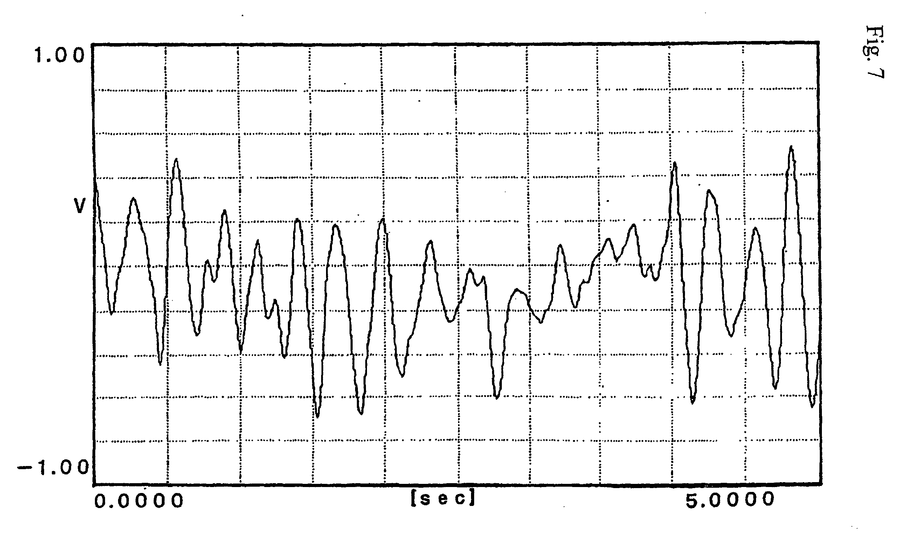

Figure 7 is a graphic representation of the waveform-analyzed seat-bottom pressure sensor output *B* when the vehicle is running with the seat occupied by a human. The output *B* contains pulses of considerable amplitude and repetition rate due both to vehicle variations, particularly vertical, and to the cushioning effect peculiar to the human body. It will nevertheless be observed that the output *B* as a whole has a sinusoidal waveform representative of human respiration. Not only respiration but also heartbeat is detectable from this sensor output by a discriminator yet to be described.

The following tabulated judgments are derivable from the various combinations of the seat-back pressure sensor output *A* and seat-bottom pressure sensor output *B* of different magnitudes, including zero. When the vehicle is at rest, the judgments are made as in Table 1 on the bases of the magnitudes of the bodily process signal components, such as those representative of human respiration and heartbeat, of the pressure sensor outputs *A* and *B.* Different reference values are set for the sensor outputs *A* and *B* because of the different cushioning capabilities of the seat back and seat bottom.

**Table 1**

| *Seat-Back Sensor* *Output A* | *Seat-Bottom Sensor* *Output B* | *Judgments* |
|---|---|---|
| more than reference *Q* | more than reference *S* | adult of average body size or more |
| more than reference *Q* | less than reference *S* | slim |
| less than reference *Q* | more than reference *S* | fat |
| less than reference *Q* more than reference *R* | less than reference *S* more than reference *T* | child |
| less than reference *R* more than zero | less than reference *T* more than zero | child seat occupied |
| zero | zero | luggage or unoccupied |

When the vehicle is traveling, on the other hand, judgments are made as in Table 2 below on the bases of not only human respiration, heartbeat and like bodily process components of the sensor outputs *A* and *B* but also the signal components representative of vehicle vibration. The vibration of the traveling vehicle provides random noise, which remains superposed on the sensor outputs *A* and *B* even after they have passed through a respiration and heartbeat filter included in the airbag control system. The reference values during vehicle travel must therefore differ from those during vehicle rest.

**Table 2**

| *Seat-Back Sensor* *Output A* | *Seat-Bottom Sensor* *Output B* | *Judgments* |
|---|---|---|
| more than reference *U* | more than reference *X* | adult of average body size or more |
| more than reference *U* | less than reference *X* | slim |
| less than reference *U* | more than reference *X* | fat |
| les than reference *U* more than reference *Y* | less than reference *X* more than reference *Y* | child |
| less than reference *V* more than reference *W* | less than reference *Y* more than reference *Z* | child seat occupied child seat occupied |
| less than reference *W* | less than reference *Z* | luggage (child seat unoccupied) |
| approx. zero | approx. zero | unoccupied |

The particular reference values for the foregoing judgments must be determined in each application of the invention depending upon such variables as vehicle seat configurations and the shape and material of the sensing bags in use. Normally, the reference values may be selected in the 35-65 range of seat occupant displacement percentage in the graph of Figure 3, where the sensor output amplitude varies steeply.

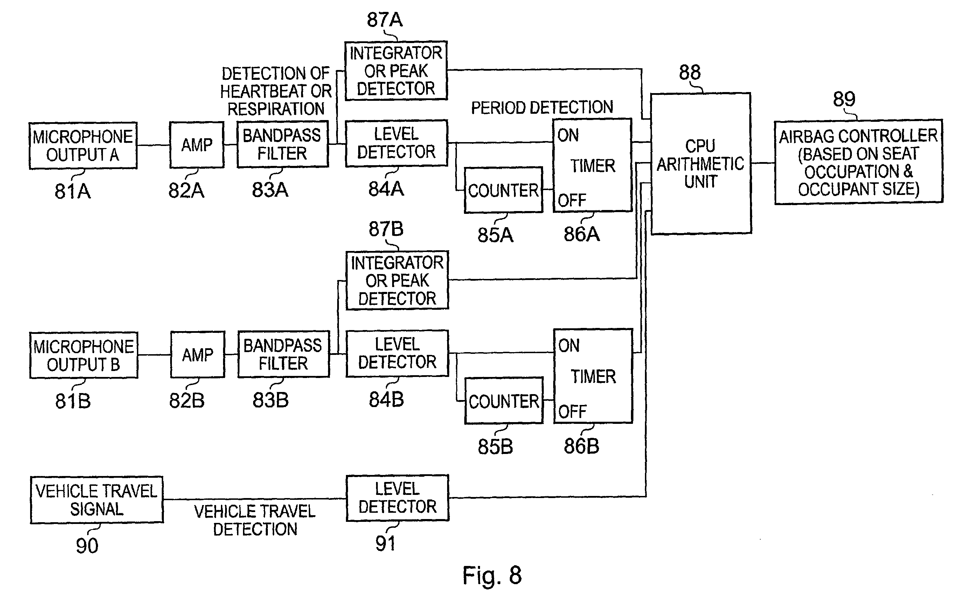

Block-diagrammatically illustrated in Figure 8 is a preferred form of airbag control system according to the invention whereby airbag performance is controlled for optimal results depending upon the particular human or nonhuman occupant, if any, of the seat as ascertained from the sensor outputs *A* and *B*. The microphones of the two pressure sensors provide these sensor outputs at 81*A* and 81*B* in Figure 8. The seat-back pressure sensor output *A* is directed through an amplifier 82*A* into a bandpass filter 83*A*. This bandpass filter may have a passband of from about 0.1 Hz to about 1.0 Hz for detection of respiration, and of from about 3 Hz to about 15 Hz for heartbeat detection.

Issuing from the bandpass filter 83*A*, the seat-back sensor output containing the desired bodily process signal component is fed into a level detector 84*A*. This level detector puts out a pulse each time the input signal rises to a predetermined level. A counter 85*A* counts the pulses from the level detector 84*A*, putting out a pulse for every two incoming pulses. A timer 86*A* is turned on by each output pulse of the level detector 84*A* and off by each output pulse of the counter 85*A*, in order to measure the duration of each period of the periodic signal.

Besides being directed into the level detector 84*A* as above, the output from the bandpass filter 83*A*, possibly representative of the seat occupant's respiration and heartbeat, is integrated or has its peak detected at 87*A*. The output from this circuit 87*A* is directed into the arithmetic unit 88 of a central processor unit (CPU) for comparison with the predetermined reference values.

Similarly, the seat-bottom pressure sensor output *B* is sequentially directed into and through an amplifier 82*B*, bandpass filter 83*B*, level detector 84*B*, counter 85*B*, timer 86*B*, and arithmetic unit 88. The output from the bandpass filter 83*B* is also fed into an integrator or peak detector 87*B* and thence into the arithmetic section 88. The arithmetic unit 88 compares the input signals from the reference values and makes the necessary judgments as in Tables 1 and 2. These judgments are utilized by an airbag controller 89 in order to generate signals for controlling the airbag accordingly.

Figure 8 further indicates at 90 a vehicle travel signal generator which puts out a signal indicative of whether the vehicle is traveling or not. The vehicle travel signal generator 90 may sense vehicle travel either mechanically or electrically, as from the rotation of some rotary part of the vehicle. Alternatively, the pressure sensor output *A* or *B* may be filtered to derive a signal component indicative of vehicle travel.

The output from the vehicle travel signal generator 90 is directed through a level detector 91 into the arithmetic unit 88. Judging from the input signal whether the vehicle is running or not, the arithmetic unit 88 switches the reference values for comparison. It is also possible to confine the airbag to one prescribed mode of operation depending upon whether the vehicle is running or at a standstill.

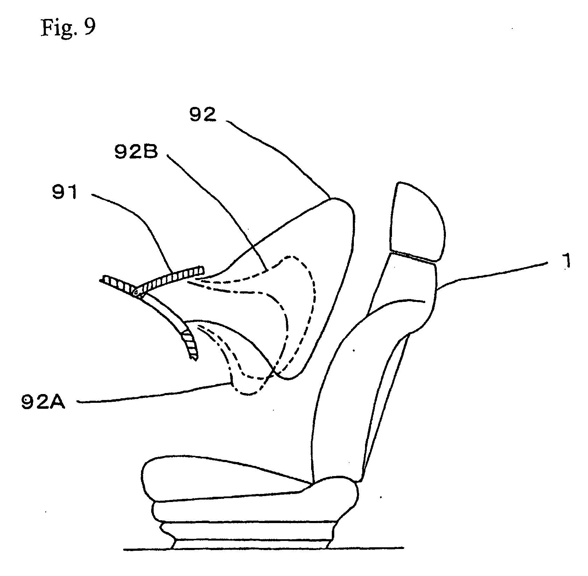

Figure 9 is explanatory of how the airbag operates under the direction of the airbag controller 89. At 1 is shown the vehicle seat in side view, in front of which there is the dashboard having a cover 91 which normally closes the airbag compartment of the dashboard. The airbag compartment cover 91 is shown open, with the airbag indicated by the solid line as inflated to the full, that is, to the conventionally standard size.

Let it be assumed that a collision has occurred while the pressure sensor outputs *A* and *B* are greater than the reference values *U* and *X,* respectively. The airbag controller 89 will then cause the airbag activator, not shown, to deploy the airbag normally, that is, fully, as indicated by the solid-line outline designated 92 in Figure 9.

If then the sensor outputs *A* and *B* are such that the arithmetic unit 88 determines that a child is seated, the airbag controller 89 will then cause the airbag to be inflated to an extent, and in a direction, fitting the child. The airbag inflated in this manner is delineated by the dot-and-dash outline 92*A* in Figure 9. For a seat occupant with a body size in between, the airbag may be inflated as depicted by the dashed outline 92*B*.

In order to control the extent to which the airbag is inflated as above, the airbag propellant may be prepared in several separate dosages, and different numbers of such propellant dosages may be detonated. The direction of airbag deployment is controllable by the angle through which the airbag compartment cover 91 is opened.

### Industrial Applicability

Thus the present invention makes it possible to determine whether the vehicle seat is occupied or not, whether the occupant is a human or luggage, and, if it is a human, what is his or her body size. The airbag is triggered or not triggered depending upon these findings, and when it is, inflated to an optimal extent and in an optimal direction for the particular seat occupant, thereby guarding the occupant from the collision without inflicting secondary damage. The invention is therefore of particular utility as a safety device of motor vehicles, particularly passenger cars.

## Claims

1. A vehicular airbag control system for controlling an airbag mounted adjacent a vehicle seat, comprising:
(a) a pneumatic pressure sensor mounted to a back of a vehicle seat for providing a bodily process signal indicative of such bodily processes as respiration and pulsation;
(b) means for comparing the magnitude of the bodily process signal with a predefined reference value; and
(c) means for controlling the extent and direction of airbag inflation according to the results of comparison of the bodily process signal with the reference value.

2. A vehicular airbag control system for controlling an airbag mounted adjacent a vehicle seat, comprising:
(a) a pneumatic pressure sensor mounted to a bottom of a vehicle seat for providing a bodily process signal indicative of such bodily processes as respiration and pulsation;
(b) means for comparing the magnitude of the bodily process signal with a predefined reference value; and
(c) means for controlling the extent and direction of airbag inflation according to the results of comparison of the bodily process signal with the reference value.

3. A vehicular airbag control system for controlling an airbag mounted adjacent a vehicle seat, comprising:
(a) a first pneumatic pressure sensor mounted to a back of a vehicle seat for providing a first bodily process signal indicative of such bodily processes as respiration and pulsation;
(b) a second pneumatic pressure sensor mounted to a bottom of a vehicle seat for providing a second bodily process signal indicative of such bodily processes as respiration and pulsation;
(c) means for comparing the magnitudes of the first and the second bodily process signal with a first and a predefined reference value, respectively; and
(d) means for controlling the extent and direction of airbag inflation according to the various combinations of the results of comparison of the first and the second bodily process signal with the first and the second reference value.

4. The airbag control system of claim 3 further comprising means for preventing the airbag from being inflated when both first and second bodily process signals are not contained in outputs from the first and the second pressure sensor.
